# EUROPÄISCHE PATENTANMELDUNG

(11) **EP 3 730 056 A1**
(43) Veröffentlichungstag der Anmeldung: **28.10.2020**
(21) Anmeldenummer: 20171314.6
(22) Anmeldetag: 24.04.2020
(51) Int. Cl.: A61B 6/03, A61B 6/04, A61N 5/10

(54) **PATIENTENBESTRAHLUNGSEINRICHTUNG**

(30) Priorität: 27.04.2019 DE 202019001877 U
(71) Anmelder: BEC GmbH, 72793 Pfullingen (DE)
(72) Erfinder: Buck, Matthias, 72764 Reutlingen (DE)
(74) Vertreter: Reinhardt, Annette

(57) **Zusammenfassung**

Eine Patientenbestrahlungseinrichtung (1) umfasst eine Patientenpositioniervorrichtung (3), eine Strahlenquelle (2) und eine Bildgebungseinrichtung (4). Die Patientenpositioniervorrichtung (3) umfasst mindestens eine Patientenaufnahme (5, 6). Die Patientenpositioniervorrichtung (3) ist zumindest zur Ausführung einer horizontalen Bewegung der Patientenaufnahme (5, 6) ausgebildet. Die Bildgebungseinrichtung (4) weist eine Abbildungseinheit (24) zur Erzeugung eines Bildes in einer Abbildungsebene (25) auf. Die Bildgebungseinrichtung (4) weist eine erste Linearbewegungseinrichtung (20) zur Bewegung der Abbildungseinheit (24) entlang einer vertikalen Bewegungsachse (21) auf. Um sowohl sitzende als auch liegende Patienten (30) abbilden zu können, ist vorgesehen, dass die Abbildungseinheit (24) um eine horizontal angeordnete Rotationsachse (22) drehbar ist.

## Beschreibung

Die Erfindung betrifft eine Patientenbestrahlungseinrichtung der im Oberbegriff des Anspruchs 1 angegebenen Gattung.

Patientenbestrahlungseinrichtungen mit einer Strahlenquelle, einer Bildgebungseinrichtung sowie einer Patientenpositioniervorrichtung sind bekannt. Die Bildgebungseinrichtung wird üblicherweise genutzt, um unmittelbar vor der Bestrahlung die genaue Position des zu bestrahlenden Gewebes im Patienten zu erfassen. Mittels der Patientenbestrahlungseinrichtung wird der Patient zunächst an der Bildgebungseinrichtung und anschließend an der Strahlenquelle positioniert. Die Abbildungseinheit erzeugt üblicherweise in einer Abbildungsebene ein Bild. Um ein größeres Volumen abzubilden, wird die Abbildungseinheit während der Abbildung bewegt, beispielsweise in vertikaler Richtung. Die Patientenbestrahlungseinrichtung steht bei bekannten Patientenbestrahlungseinrichtungen während der Abbildung still. Mit einer solchen vertikal beweglichen Abbildungseinheit können sitzende Patienten gut abgebildet werden.

Der Erfindung liegt die Aufgabe zugrunde, eine Patientenbestrahlungseinrichtung der gattungsgemäßen Art zu schaffen, die einfach aufgebaut ist und vielfältig einsetzbar ist.

Diese Aufgabe wird durch eine Patientenbestrahlungseinrichtung mit den Merkmalen des Anspruchs 1 gelöst.

Die Erfindung sieht vor, dass die Abbildungseinheit um eine horizontal angeordnete Rotationsachse drehbar ist. Dadurch kann auch ein liegend angeordneter Patient mit der

Bildgebungseinrichtung abgebildet werden, wenn die Abbildungseinheit so angeordnet wird, dass die Abbildungsebene vertikal steht und der Patient während der Abbildung horizontal gegenüber der Abbildungseinheit bewegt wird. Für die horizontale Bewegung des Patienten gegenüber der Abbildungseinheit wird vorzugsweise die Patientenpositioniervorrichtung genutzt. Eine Linearbewegungseinrichtung, die die Abbildungseinheit in horizontaler Richtung bewegt, kann dadurch entfallen, so dass sich ein einfacher Aufbau des Systems ergibt. Aufgrund der Drehbarkeit der Abbildungseinheit in Verbindung mit der Möglichkeit einer horizontalen Bewegung des Patienten durch die Patientenpositioniervorrichtung können auf einfache Weise sowohl sitzende als auch liegende Patienten abgebildet werden.

Als Linearbewegungseinrichtung wird vorliegend eine Bewegungseinrichtung bezeichnet, die ausschließlich zur Ausführung einer linearen Bewegung in einer Richtung ausgebildet ist. Die Linearbewegungseinrichtung besitzt demnach genau einen translatorischen und keinen rotatorischen Freiheitsgrad der Bewegung. Die Linearbewegungseinrichtung ist vorzugsweise eine Linearachse.

Vorzugsweise ist vorgesehen, dass die Patientenpositioniervorrichtung einen Roboterarm umfasst. Der Roboterarm ist vorteilhaft mittels einer zweiten Linearbewegungseinrichtung entlang einer horizontalen Bewegungsachse bewegbar. Der Roboterarm ist dabei vorteilhaft an einem angetriebenen Schlitten der zweiten Linearbewegungseinrichtung fixiert. Alternativ kann auch vorgesehen sein, dass der Roboterarm selbst eine lineare, horizontale Bewegung der Patientenaufnahme mit ausreichender Genauigkeit ausführen kann. In diesem Fall kann eine zweite Linearbewegungseinrichtung auch entfallen.

Der Roboterarm weist vorzugsweise mindestens sechs Rotationsachsen, also sechs voneinander unabhängige Achsen, um die Abschnitte des Roboterarms zueinander verschwenkt werden können, auf. Ein solcher sogenannter Sechsachsroboter erlaubt eine freie Positionierung und Verschwenkung der an dem Roboterarm gehaltenen Patientenaufnahme. Es ist eine genaue und schnelle Positionierung des Patienten möglich. Die Patientenaufnahme ist an dem Roboterarm vorzugsweise abnehmbar gehalten. Vorteilhaft besitzt die Patientenpositioniervorrichtung eine erste Patientenaufnahme in Form eines Sitzes und eine zweite Patientenaufnahme in Form einer Liege, die wahlweise an dem Roboterarm angeordnet werden können.

Eine vorteilhafte Anordnung ergibt sich, wenn der Roboterarm hängend an der ersten Linearbewegungseinrichtung angeordnet ist. Die Linearbewegungseinrichtung ist vorzugsweise an der Decke des Raums, in dem die Patientenbestrahlungseinrichtung angeordnet ist, gehalten, und der Roboterarm ist mit seinem Ständer hängend an der Linearbewegungseinrichtung angeordnet. Dadurch ist der Boden für andere Einrichtungen frei zugänglich, und es kann ein großer Bewegungsbereich für die Patientenaufnahme erzielt werden, ohne dass die Nutzung des Raums durch Einbauten am Boden eingeschränkt wird.

Die Bildgebungseinrichtung und die Strahlenquelle sind vorzugsweise über eine Abschirmwand voneinander trennbar. Viele bekannte Bildgebungseinrichtungen sind strahlungsempfindlich. Die Strahlung der Strahlenquelle führt zu Verschleiß und Alterung der Teile der Bildgebungseinrichtung. Über die räumliche Trennung der Bildgebungseinrichtung von der Strahlenquelle kann die Strahlenexposition der Bildgebungseinrichtung minimiert werden. Die Abschirmwand ist vorzugsweise zu öffnen, so dass zwischen der Bildgebungseinrichtung und der Strahlenquelle ein Durchgang für die Patientenaufnahme gebildet werden kann. Das Öffnen der Abschirmwand erfolgt vorzugsweise automatisiert. Die Bewegung des Patienten aus dem Raumteil, in dem die Strahlenquelle angeordnet ist, in den von der Abschirmwand abgeteilten Raumteil, in dem die Bildgebungseinrichtung angeordnet ist, erfolgt vorzugsweise über eine kombinierte Bewegung des Roboterarms und der ersten Linearbewegungseinrichtung.

Ein einfacher, ansprechender Aufbau ergibt sich, wenn die Abbildungseinheit ringförmig ausgebildet ist. Ein liegender Patient wird während der Abbildung vorzugsweise von der Patientenpositioniervorrichtung gegenüber der ringförmigen Abbildungseinheit bewegt. Bei einem sitzenden Patienten ist vorzugsweise vorgesehen, dass sich die Abbildungseinheit während der Abbildung in vertikaler Richtung gegenüber dem Patienten bewegt. Mindestens eine Linearbewegungseinrichtung ist vorteilhaft eine Linearachse. Insbesondere sind die erste Linearbewegungseinrichtung und die zweite Linearbewegungseinrichtung Linearachsen.

Vorzugsweise weist die Abbildungseinheit genau zwei Bewegungsachsen, nämlich die vertikale Bewegungsachse und die Rotationsachse, auf. Weitere Bewegungsachsen, die lineare Bewegungen oder Rotationsbewegungen der Abbildungseinheit im Raum ermöglichen, sind vorzugsweise nicht vorgesehen. Dadurch ergibt sich ein einfacher Aufbau der Abbildungseinheit.

Ein Ausführungsbeispiel der Erfindung wird im Folgenden anhand der schematischen Zeichnung erläutert. Es zeigen:
- Fig. 1: eine perspektivische Ansicht eines ersten Raumteils, in dem die Patientenpositioniervorrichtung und die Strahlenquelle angeordnet sind,
- Fig. 2: eine perspektivische Darstellung eines zweiten Raumteils, in dem die Bildgebungseinrichtung angeordnet ist,
- Fig. 3: eine Seitenansicht der Patientenbestrahlungseinrichtung in einer Anordnung vor einer Abbildung eines sitzenden Patienten,
- Fig. 4: eine Seitenansicht während einer Abbildung eines nicht dargestellten, sitzenden Patienten,
- Fig. 5: eine perspektivische Darstellung der Patientenbestrahlungseinrichtung während der Bewegung eines sitzenden Patienten von der Bildgebungseinrichtung zur Strahlenquelle,
- Fig. 6: eine perspektivische Darstellung der Patientenbestrahlungseinrichtung in einer Anordnung für die Bestrahlung eines sitzenden Patienten,
- Fig. 7: die Patientenbestrahlungseinrichtung in Seitenansicht beim Transport eines liegenden Patienten,
- Fig. 8: eine perspektivische Darstellung des zweiten Raumteils beim Transport des Patienten zur Bildgebungseinrichtung,
- Fig. 9: eine Seitenansicht der Patientenbestrahlungseinrichtung während der Abbildung eines liegenden Patienten,
- Fig. 10: eine perspektivische Darstellung der Patientenbestrahlungseinrichtung beim Transport eines liegenden Patienten von der Bildgebungseinrichtung zur Strahlenquelle und
- Fig. 11: eine Seitenansicht der Patientenbestrahlungseinrichtung in einer Position während der Bestrahlung eines liegenden Patienten.

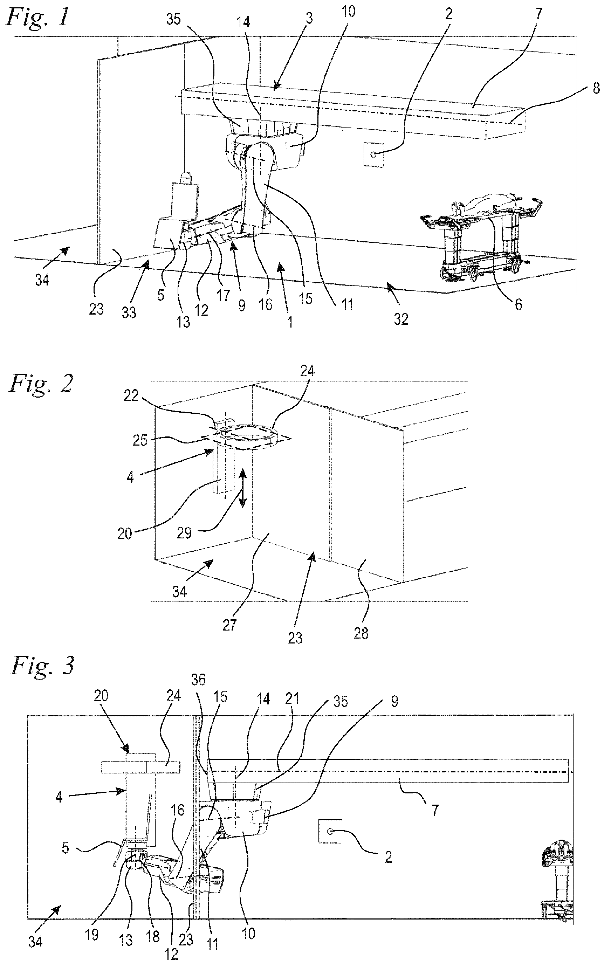

Fig. 1 zeigt schematisch eine Patientenbestrahlungseinrichtung 1. Die Patientenbestrahlungseinrichtung 1 ist in einem Raum 32 angeordnet, der über eine Abschirmwand 23 in zwei Raumteile 33 und 34 geteilt ist. Fig. 1 zeigt den ersten Raumteil 33, in dem die Bestrahlung eines Patienten erfolgt, im Einzelnen, während der zweite Raumteil 34 weitgehend von der Abschirmwand 23 verdeckt ist. In dem ersten Raumteil 33 mündet eine Strahlenquelle 2. Die Strahlenquelle 2 kann beispielsweise eine Quelle für Protonen, Neutronen, Ionen oder dergleichen sein. Die Strahlenquelle 2 kann auch einen Photonenstrahl wie einen Röntgen- oder Gammastrahl erzeugen.

Um Patienten an der Strahlenquelle 2 exakt positionieren zu können, ist eine Patientenpositioniervorrichtung 3 vorgesehen. Die Patientenpositioniervorrichtung 3 umfasst eine erste Linearbewegungseinrichtung 7, im Ausführungsbeispiel eine von einem nicht dargestellten Elektromotor angetriebene Linearachse, an der ein Roboterarm 9 gehalten ist. Die Linearachse weist einen einzigen translatorischen Bewegungsfreiheitsgrad und keinen rotatorischen Bewegungsfreiheitsgrad auf. Der Roboterarm 9 kann über die Linearbewegungseinrichtung 7 entlang einer horizontal verlaufenden Bewegungsachse 8 bewegt werden. Die Linearbewegungseinrichtung 7 ist bevorzugt an einer nicht dargestellten Decke des Raums 32 fixiert. Anstatt des Roboterarms 9 kann auch eine anders gestaltete Bewegungseinrichtung, beispielsweise eine Anordnung mit mehreren Linearbewegungseinrichtungen, vorgesehen sein. Am Roboterarm 9 ist in der Darstellung in Fig. 1 eine erste Patientenaufnahme 5 gehalten, die als Sitz mit Fußabstützung, Sitzfläche und Rückenlehne ausgebildet ist. Alternativ kann am Roboterarm 6 auch eine zweite Patientenaufnahme 6 angeordnet werden, die als Liege ausgebildet ist und im Ausführungsbeispiel flach und horizontal verläuft.

Der Roboterarm 9 ist im Ausführungsbeispiel als konventioneller Sechsachsroboter ausgebildet. Der Roboterarm 9 besitzt einen Ständer 35, der von der ersten Linearbewegungseinrichtung 7 entlang der Bewegungsachse 8 beweglich ist. Am Ständer 35 ist ein Karussell 10 um eine erste Rotationsachse 14 drehbar gelagert. Die erste Rotationsachse 14 ist vertikal und senkrecht zur Bewegungsachse 8 angeordnet. Am Karussell 10 ist eine Schwinge 11 um eine zweite Rotationsachse 15 schwenkbar gelagert. Die zweite Rotationsachse 15 verläuft horizontal. An dem dem Karussell 10 abgewandten Ende der Schwinge 11 ist ein Arm 12 angeordnet, der um eine dritte Rotationsachse 16 schwenkbar gelagert ist. Die dritte Rotationsachse 16 verläuft parallel zur zweiten Rotationsachse 15. An dem der Schwinge 11 abgewandten Ende des Arms 12 ist eine Hand 13 um eine vierte Rotationsachse 17 drehbar gelagert. Die vierte Rotationsachse 17 verläuft etwa in Längsrichtung des Arms 12. Die Hand 13 besitzt eine fünfte Rotationsachse 18 sowie eine sechste Rotationsachse 19, die in Fig. 3 schematisch dargestellt sind. Aufgrund der sechs Rotationsachsen 14 bis 19 kann die Patientenaufnahme 5 an der Hand 13 Bewegungen in allen drei Raumrichtungen und Drehungen um alle drei Raumachsen durchführen. Dadurch, dass der Roboterarm 9 hängend angeordnet ist, bleibt der Boden frei.

Fig. 2 zeigt den zweiten Raumteil 34. Im zweiten Raumteil 34 ist eine Bildgebungseinrichtung 4, beispielsweise ein Computertomograph angeordnet. Die Bildgebungseinrichtung 4 umfasst eine Abbildungseinheit 24, die eine im Ausführungsbeispiel nicht dargestellte Röntgenquelle sowie einen Röntgendetektor aufweisen kann. Mit der Abbildungseinheit 24 kann eine Abbildung des Patienten in einer Abbildungsebene 25 erzeugt werden. Die Abbildungseinheit 24 ist ringförmig und die Abbildungsebene 25 liegt senkrecht zur Mittelachse des die Abbildungseinheit 24 bildenden Rings. Die Abbildungseinheit 24 ist an einer zweiten Linearbewegungseinrichtung 20 gehalten. Die zweite Linearbewegungseinrichtung 20 ermöglicht eine Bewegung der Abbildungseinheit 24 in Richtung einer vertikal angeordneten Bewegungsachse 21. Dies ist durch den Doppelpfeil 29 in Fig. 2 verdeutlicht. Die Abbildungseinheit 24 ist außerdem um eine Rotationsachse 22 drehbar an der zweiten Linearbewegungseinrichtung 20 gehalten. Die Rotationsachse 22 verläuft horizontal und im Ausführungsbeispiel senkrecht zu der Wand, an der die Linearbewegungseinrichtung 20 angeordnet ist. Die Bildgebungseinrichtung 4 besitzt im Ausführungsbeispiel ausschließlich zwei Bewegungsachsen, nämlich die lineare Bewegungsachse 21 sowie die Rotationsachse 22. Eine horizontale Linearbewegung kann die Abbildungseinheit 24 nicht ausführen.

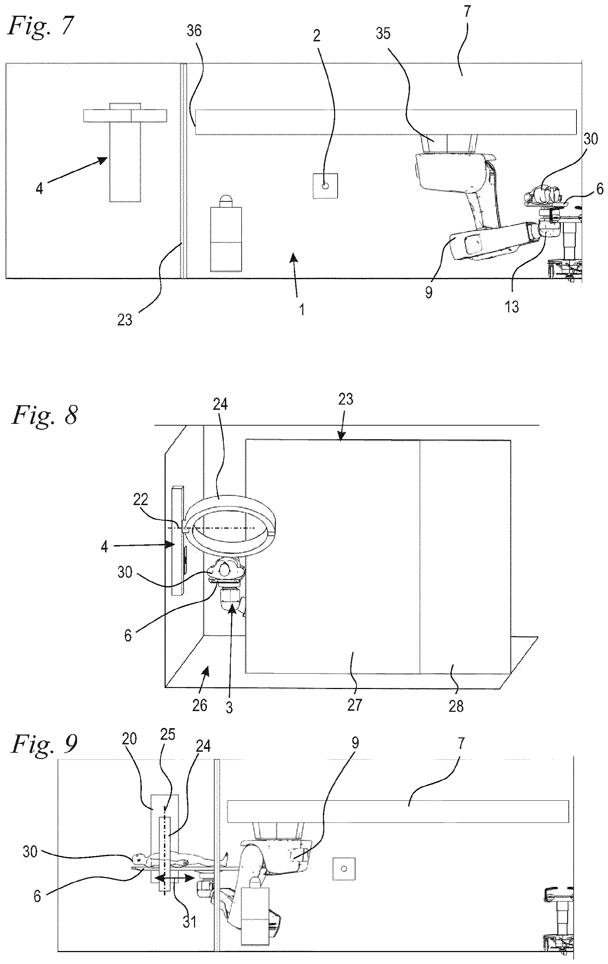

Wie Fig. 2 auch zeigt, besitzt die Abschirmwand 23 im Ausführungsbeispiel zwei Wandabschnitte 27 und 28. Der erste Wandabschnitt 27 ist beweglich angeordnet und kann einen in Fig. 8 sichtbaren Durchgang 26 freigeben.

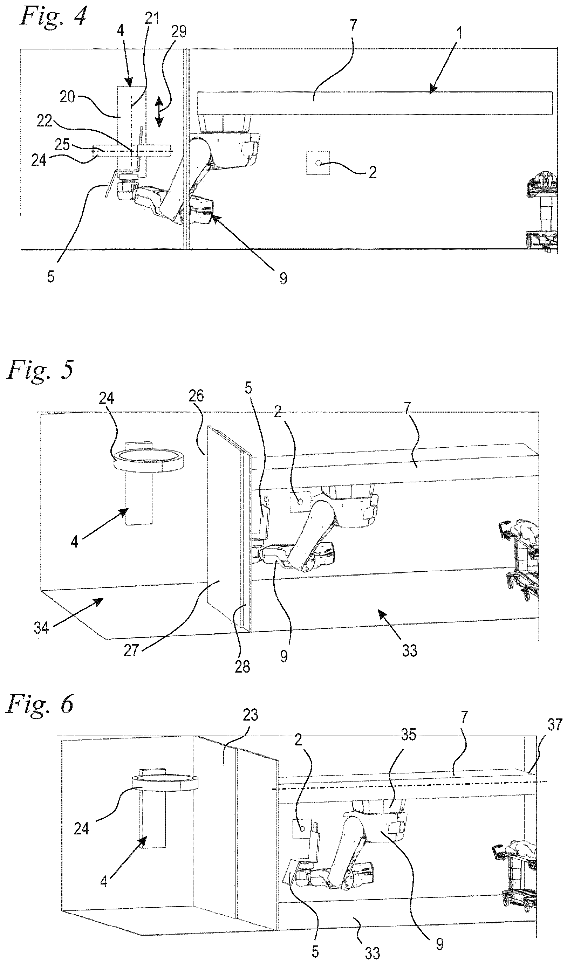

Bei der in Fig. 3 dargestellten Seitenansicht ist der Durchgang 26 (Fig. 8) geöffnet. Der Roboterarm 6 ragt durch den Durchgang 26. Die Patientenaufnahme 5 ist an der Bildgebungseinrichtung 4 im zweiten Raumteil 34 angeordnet. Im Ausführungsbeispiel befindet sich der Ständer 35 des Roboterarms 9 in dieser Position nahe eines ersten Endes 36 der ersten Linearbewegungseinrichtung 7. Das erste Ende 36 ist das der Abschirmwand 23 zugewandte Ende der ersten Linearbewegungseinrichtung 7. In der in Fig. 3 dargestellten Position kann von einem auf der Patientenaufnahme 5 angeordneten, nicht dargestellten Patienten eine Abbildung mit der Bildgebungseinrichtung 4 erzeugt werden. Hierzu wird die Abbildungseinheit 24 wie in Fig. 4 dargestellt nach unten über den Patienten bewegt und anschließend, wie durch den Doppelpfeil 29 angedeutet, wieder zurück bewegt. Die Abbildungseinheit 24 befindet sich dabei in einer Lage, in der die Bildgebungsebene 25 horizontal verläuft.

Wenn die Abbildungseinheit 24 aus dem Bereich des Patienten bewegt wurde, kann der Patient aus dem zweiten Raumteil 34 durch den Durchgang 26 zurück in den ersten Raumteil 33 bewegt werden. Fig. 5 zeigt die Anordnung, wenn sich die Patientenaufnahme 5 wieder im ersten Raumteil 33 befindet. Der Wandabschnitt 27 bewegt sich wieder zurück in seine Ausgangsposition, um den Durchgang 26 zu verschließen. Anschließend oder währenddessen wird der Patient im Raumteil 33 an der Strahlenquelle 2 positioniert. Die Positionierung erfolgt anhand der über die Bildgebungseinrichtung 4 erzeugten Abbildung des zu bestrahlenden Gewebes. Für die Positionierung des Patienten an der Strahlenquelle 2 wird der Ständer 35 des Roboterarms 9 von der Linearbewegungseinrichtung 7 in Richtung der Bewegungsachse 8 vom ersten Ende 36 in Richtung auf ein zweites Ende 37 der Linearbewegungseinrichtung 7 bewegt. Bei geschlossener Abschirmwand 23 wird die Bestrahlung des Patienten durchgeführt. Dadurch, dass die Abschirmwand 23 geschlossen ist, ist die Bildgebungseinrichtung 4 vor den Strahlen geschützt. Von der Erzeugung der Abbildung bis zum Ende der Bestrahlung befindet sich der Patient vorzugsweise unbeweglich auf der Patientenaufnahme, so dass eine genaue Positionierung möglich ist.

Anstatt eines sitzenden Patienten kann mit der Patientenbestrahlungseinrichtung 1 auch ein liegender Patient behandelt werden. In Fig. 7 ist schematisch ein Patient 30 auf einer zweiten Patientenaufnahme 6 dargestellt, die als Liege ausgebildet ist. Der Roboterarm 9 kann die Liege 6 aufnehmen. Hierzu wird die Hand 13 an der Unterseite der Patientenaufnahme 6 befestigt. Die Befestigung kann automatisiert oder manuell über einen Bediener erfolgen. Wie Fig. 8 zeigt, wird anschließend die Abschirmwand 23 durch Bewegung des ersten Wandabschnitts 27 geöffnet, und der Patient 30 wird mit der Patientenpositioniervorrichtung 3 zur Bildgebungseinrichtung 4 bewegt. Dabei bewegt sich der Ständer 35 an der Linearbewegungseinrichtung 7 in Richtung zum ersten Ende 36 (Fig. 7). Um einen liegenden Patienten 30 abzubilden, wird die Abbildungseinheit 24 um die Rotationsachse 22 um 90° gedreht, so dass die Abbildungsebene 25 vertikal verläuft. Dies ist in Fig. 8 schematisch dargestellt. Die Abbildungseinheit 24 kann außerdem auf eine gewünschte Höhe bewegt werden. Anschließend wird der Patient 30 in horizontaler Richtung gegenüber der Abbildungseinheit 24 bewegt. Die horizontale Bewegung des Patienten 30 erfolgt über den Roboterarm 9 und/oder die erste Linearbewegungseinrichtung 7. Eine horizontale Bewegung der Abbildungseinheit 24 ist nicht vorgesehen. Während der Abbildung steht die Abbildungseinheit 24 still.

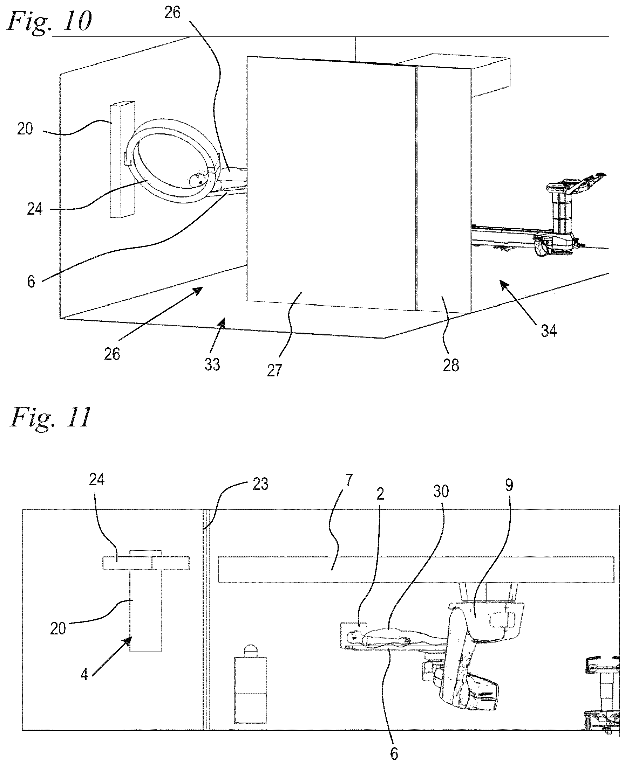

Nach Erstellung der Abbildung wird der Patient 30 wie in Fig. 10 schematisch dargestellt durch den Durchgang 26 in den zweiten Raumteil 34 bewegt. Diese Bewegung kann vorteilhaft über eine kombinierte Bewegung des Roboterarms 9 und der ersten Linearbewegungseinrichtung 7 erfolgen. Anschließend wird die Abschirmwand 23 durch Bewegung des ersten Wandabschnitts 27 verschlossen, so dass die Bildgebungseinrichtung 4 vor Strahlung geschützt ist. Damit wird der Patient 30 an der Strahlenquelle 2 positioniert, wie in Fig. 11 dargestellt ist, und die Bestrahlung erfolgt.

In alternativer Ausführung kann vorgesehen sein, dass die Patientenpositioniervorrichtung 3 keine Linearbewegungseinrichtung aufweist. Die horizontale Bewegung der Patientenaufnahme 5, 6 kann bei dieser Ausführung vorteilhaft durch den Roboterarm 6 ausgeführt werden. Hierzu ist ein Roboterarm 6 vorgesehen, der eine translatorische horizontale Bewegung mit ausreichender Genauigkeit durch Interpolation von rotatorischen Bewegungen ausführt.

## Patentansprüche

1. Patientenbestrahlungseinrichtung umfassend eine Patientenpositioniervorrichtung (3), eine Strahlenquelle (2) und eine Bildgebungseinrichtung (4), wobei die Patientenpositioniervorrichtung (3) mindestens eine Patientenaufnahme (5, 6) umfasst, wobei die Patientenpositioniervorrichtung (3) zumindest zur Ausführung einer horizontalen Bewegung der Patientenaufnahme (5, 6) ausgebildet ist, wobei die Bildgebungseinrichtung (4) eine Abbildungseinheit (24) zur Erzeugung eines Bilds in einer Abbildungsebene (25) aufweist und wobei die Bildgebungseinrichtung (4) eine erste Linearbewegungseinrichtung (20) zur Bewegung der Abbildungseinheit (24) entlang einer vertikalen Bewegungsachse (21) aufweist,
**dadurch gekennzeichnet, dass** die Abbildungseinheit (24) um eine horizontal angeordnete Rotationsachse (22) drehbar ist.

2. Patientenbestrahlungseinrichtung nach Anspruch 1,
**dadurch gekennzeichnet, dass** die Patientenpositioniervorrichtung (3) einen Roboterarm (9) umfasst.

3. Patientenbestrahlungseinrichtung nach Anspruch 2,
**dadurch gekennzeichnet, dass** der Roboterarm (9) mittels einer zweiten Linearbewegungseinrichtung (7) entlang einer horizontalen Bewegungsachse (8) bewegbar ist.

4. Patientenbestrahlungseinrichtung nach Anspruch 2 oder 3,
**dadurch gekennzeichnet, dass** der Roboterarm (9) mindestens sechs Rotationsachsen (14, 15, 16, 17, 18 ,19) aufweist.

5. Patientenbestrahlungseinrichtung nach Anspruch 3 oder 4,
**dadurch gekennzeichnet, dass** die Patientenaufnahme (5, 6) an dem Roboterarm (9) abnehmbar gehalten ist.

6. Patientenbestrahlungseinrichtung nach einem der Ansprüche 3 bis 5,
**dadurch gekennzeichnet, dass** die Patientenpositioniervorrichtung (3) eine erste Patientenaufnahme (5) in Form eines Sitzes und eine zweite Patientenaufnahme (6) in Form einer Liege aufweist, die wahlweise an dem Roboterarm (6) angeordnet werden können.

7. Patientenbestrahlungseinrichtung nach einem der Ansprüche 3 bis 6,
**dadurch gekennzeichnet, dass** der Roboterarm (9) hängend an der ersten Linearbewegungseinrichtung (7) angeordnet ist.

8. Patientenbestrahlungseinrichtung nach einem der Ansprüche 1 bis 7,
**dadurch gekennzeichnet, dass** die Bildgebungseinrichtung (4) und die Strahlenquelle (2) über eine Abschirmwand (23) voneinander trennbar sind.

9. Patientenbestrahlungseinrichtung nach Anspruch 8,
**dadurch gekennzeichnet, dass** die Abschirmwand (23) zu öffnen ist, so dass zwischen der Bildgebungseinrichtung (4) und der Strahlenquelle (2) ein Durchgang (26) für die Patientenaufnahme (5, 6) gebildet werden kann.

10. Patientenbestrahlungseinrichtung nach einem der Ansprüche 1 bis 9,
**dadurch gekennzeichnet, dass** die Abbildungseinheit (24) ringförmig ausgebildet ist.

11. Patientenbestrahlungseinrichtung nach einem der Ansprüche 1 bis 10,
**dadurch gekennzeichnet, dass** mindestens eine Linearbewegungseinrichtung (7, 20) eine Linearachse ist.

12. Patientenbestrahlungseinrichtung nach Anspruch 11,
**dadurch gekennzeichnet, dass** die Abbildungseinheit (24) genau zwei Bewegungsachsen, nämlich die vertikale Bewegungsachse (20) und die Rotationsachse (22), aufweist.
